# EUROPÄISCHE PATENTSCHRIFT

(11) **EP 2 867 337 B1**
(45) Veröffentlichungstag und Bekanntmachung des Hinweises auf die Patenterteilung: **04.11.2015**
(21) Anmeldenummer: 13745591.1
(22) Anmeldetag: 06.08.2013
(51) Int. Cl.: C10G 9/36

(54) **VERFAHREN ZUR HERSTELLUNG VON OLEFINEN DURCH THERMISCHES DAMPFSPALTEN IN SPALTÖFEN**
METHOD FOR PRODUCING OLEFINS BY MEANS OF THERMAL STEAM CRACKING IN CRACKING FURNACES
PROCÉDÉ DE FABRICATION D'OLÉFINES PAR CRAQUAGE THERMIQUE À LA VAPEUR D'EAU DANS DES FOURS DE CRAQUAGE

(30) Priorität: 09.08.2012 EP 12005782
(43) Veröffentlichungstag der Anmeldung: 06.05.2015
(73) Patentinhaber: Linde Aktiengesellschaft, 80331 München (DE)
(72) Erfinder: SCHMIDT, Gunther, 82041 Deisenhofen (DE); FRITZ, Helmut, 81375 München (DE); WALTER, Stefanie, 82418 Seehausen (DE)
(86) Internationale Anmeldenummer: PCT/EP2013/002347
(87) Internationale Veröffentlichungsnummer: WO 2014/023417

(56) Entgegenhaltungen:
- US-A- 2 017 874
- US-A1- 2004 209 964
- US-A1- 2008 194 900
- US-A1- 2008 223 754
- US-B2- 6 743 961

## Beschreibung

Die vorliegende Erfindung betrifft ein Verfahren zur Umsetzung von Kohlenwasserstoffeinsätzen durch thermisches Dampfspalten zu mindestens einem olefinhaltigen Produktstrom, welcher zumindest Ethylen und Propylen enthält, wobei ein erster Kohlenwasserstoffeinsatz in mindestens einem ersten Spaltofen und ein zweiter Kohlenwasserstoffeinsatz in mindesten einem zweiten Spaltofen wenigstens teilweise umgesetzt wird.

Beim thermischen Dampfspalten (auch als Dampfcracken oder Steamcracken bezeichnet, engl. Steam Cracking) handelt es sich um ein seit langem etabliertes Verfahren der Petrochemie. Die klassische Zielverbindung beim thermischen Dampfspalten ist das Ethylen (auch: Ethen), das eine wichtige Ausgangsverbindung für eine Reihe chemischer Synthesen darstellt.

Als Einsatz für das thermische Dampfspalten können sowohl Gase wie Ethan, Propan oder Butan und entsprechende Gemische als auch flüssige Kohlenwasserstoffe, wie beispielsweise Naphtha, und Kohlenwasserstoffgemische verwendet werden.

Zu den beim thermischen Dampfspalten im Einzelnen verwendeten Vorrichtungen und Reaktionsbedingungen und zu den ablaufenden Reaktionen sowie zu Einzelheiten der Raffinerietechnik sei auf entsprechende Artikel in Nachschlagewerken wie Zimmermann, H. und Walzl, R.: Ethylene. In: Ullmann's Encyclopedia of Industrial Chemistry. 6. Aufl. Weinheim: Wiley-VCH, 2005, und Irion, W.W. und Neuwirth, O.S.: Oil Refining. In: Ullmann's Encyclopedia of Industrial Chemistry. 6. Aufl. Weinheim: Wiley-VCH 2005, verwiesen. Verfahren zur Herstellung von Olefinen sind beispielsweise auch in der US 3 714 282 A und der US 6 743 961 B1 offenbart, welche ein Verfahren zur Herstellung von Olefinen beinhaltet, bei welchem Schweröl in einem milden Spaltprozess vorbehandelt wird bevor es in einen Ofen zum thermische Spalten geführt wird.

Desweitern sei hier die US 2004/209964 erwähnt, welche offenbart, dass Kohlenwasserstoffe mit einer Kohlenstoffzahl zwischen 15 und 30, die über eine Fischer-Tropsch-Synthese hergestellt und fraktioniert wurden, nach einer Hydrierung mild thermisch gespalten werden.

Zum thermischen Dampfspalten werden Spaltöfen eingesetzt. Die Spaltöfen sind, zusammen mit Quechenheit und nachgeschalteten Einrichtungen zur Aufarbeitung der gebildeten Produktgemische, in entsprechenden größeren Anlagen zur Olefinherstellung integriert, die im Rahmen dieser Anmeldung als "Steamcracker" bezeichnet werden.

Eine wichtige Kenngröße beim thermischen Dampfspalten ist die sogenannte Spaltschärfe (engl. Cracking Severity), welche die Spaltbedingungen bestimmt. Die Spaltbedingungen werden insbesondere beeinflusst von der Temperatur und der Verweilzeit sowie der Partialdrücke der Kohlenwasserstoffe und des Wasserdampfs. Auch die Zusammensetzung der als Einsatz verwendeten Kohlenwasserstoffgemische und die Bauart der verwendeten Spaltöfen beeinflussen die Spaltbedingungen. Aufgrund der wechselseitigen Einflüsse dieser Faktoren wird die Spaltbedingung normalerweise über das Verhältnis von Propylen (auch als Propen bezeichnet) zu Ethylen im Spaltgas festgelegt.

Beim thermischen Dampfspalten entstehen je nach Einsatzgemisch und Spaltbedingungen neben der klassischen Zielverbindung Ethylen mitunter beträchtliche Mengen an Nebenprodukten, die aus einem entsprechenden Produktstrom abgetrennt werden können. Hierbei handelt es sich unter anderem um niedere Alkene wie z.B. Propylen und Butene sowie Diene, wie beispielsweise Butadiene, und sowie Aromaten wie z.B. Benzol, Toluol und Xylole. Diese besitzen einen vergleichsweise hohen wirtschaftlichen Wert, so dass ihre Bildung als sogenannte Mehrwertprodukte (engl. High Value Products) erwünscht ist.

Die US 2008/0194900 A1 offenbart ein Verfahren zur Erzeugung von Olefinen durch Dampfspaltung von aromatischem Naphtha, bei dem gebildetes Ethan, Propan und ein gebildeter C₅-Olefinstrom in die Spaltung zurückgeführt werden können.

Die US 6 743 961 B2 offenbart ein Verfahren zur Erzeugung von Olefinen, bei dem Rohöl in einer kombinierten Verdampfungs- und Spalteinheit teilweise verdampft wird. Der gebildete Dampf und die verbleibende Flüssigkeit werden bei unterschiedlichen Spaltbedingungen gespalten.

In der US 2004/209964 A1 wird ein Verfahren vorgeschlagen, bei dem ein Fischer-Tropsch-Produktstrom fraktioniert wird. Kohlenwasserstoffe unterschiedlicher Kettenlängen werden bei unterschiedlichen Spaltbedingungen gespalten.

Die vorliegende Erfindung stellt sich die Aufgabe, die Möglichkeiten zur Gewinnung von olefinhaltigen Produktgemischen aus Kohlenwasserstoffen durch thermisches Dampfspalten zu verbessern.

### Offenbarung der Erfindung

Die Erfindung schlägt vor diesem Hintergrund ein Verfahren zur Umsetzung von Kohlenwasserstoffeinsätzen durch thermisches Dampfspalten zu einem olefinhaltigen Produktstrom, welcher zumindest Ethylen und Propylen enthält, wobei ein erster Kohlenwasserstoffeinsatz in mindestens einem ersten Spaltofen und ein zweiter Kohlenwasserstoffeinsatz in mindesten einem zweiten Spaltofen wenigstens teilweise umgesetzt wird, mit den Merkmalen der unabhängigen Patentansprüche vor. Bevorzugte Ausgestaltungen sind Gegenstand der Unteransprüche und der nachfolgenden Beschreibung.

### Vorteile der Erfindung

Erfindungsgemäß wird ein Verfahren vorgeschlagen, bei welchem der zweite Kohlenwasserstoffeinsatz im zweiten Spaltofen mit Spaltbedingungen umgesetzt wird, die zu einem Verhältnis von Propylen zu Ethylen von 0,85 bis 1,6 kg/kg, und der erste Kohlenwasserstoffeinsatz im ersten Spaltofen mit Spaltbedingungen umgesetzt wird, die zu einem Verhältnis von Propylen zu Ethylen von 0,25 bis 0,85 kg/kg am Spaltofenaustritt führen, wobei der Wert für das Verhältnis von Propylen zu Ethylen für den zweiten Kohlenwasserstoffeinsatz über dem Wert für das Verhältnis von Propylen zu Ethylen für den ersten Kohlenwasserstoffeinsatz liegt. Dabei unterscheiden sich erster und zweiter Kohlenwasserstoffeinsatz in ihrer Zusammensetzung.

Im Rahmen der Erfindung werden als erster beziehungsweise zweiter Kohlenwasserstoffeinsatz alle Kohlenwasserstoffe bezeichnet, die in den ersten beziehungsweise in den zweiten Spaltofen geführt werden. Es wird also ein erster Kohlenwasserstoffeinsatz in einem ersten Spaltofen zumindest teilweise umgesetzt und ein zweiter Kohlenwasserstoffeinsatz in einem zweiten Spaltofen und zwar jeweils bei Spaltbedingungen, wie sie in Anspruch 1 angegeben sind. Spaltbedingungen, wie sie im zweiten Spaltofen herrschen und wie sie in Anspruch 1 mit dem entsprechenden Verhältnis von Propylen zu Ethylen angegeben sind, werden im Folgenden als milde Spaltbedingungen bezeichnet, während Spaltbedingungen, wie sie im ersten Spaltofen herrschen und welche ebenfalls in Anspruch 1 durch das Verhältnis von Propylen zu Ethylen angegeben sind, werden im Folgenden als normale Spaltbedingungen bezeichnet. Normale Spaltbedingungen sind Spaltbedingungen, wie sie üblicherweise bei den thermischen Dampfspalten verwendet werden.

Unter Spaltofen wird im Rahmen dieser Erfindung eine Spalteinheit verstanden, in der die Spaltbedingungen festgelegt sind. Es ist möglich, dass an einem Gesamtofen eine Unterteilung in zwei oder mehr Spaltöfen vorliegt. Man spricht dann häufig von Ofenzellen. Mehrere, zu einem Gesamtofen gehörende Ofenzellen weisen in der Regel voneinander unabhängige Strahlungszonen und eine gemeinsame Konvektionszone sowie einen gemeinsamen Rauchabzug auf. In diesen Fällen kann jede Ofenzelle mit eigenen Spaltbedingungen betrieben werden. Jede Ofenzelle ist somit eine Spalteinheit und wird infolgedessen hier als Spaltofen bezeichnet. Der Gesamtofen weist dann mehrere Spalteinheiten oder, anders ausgedrückt, er weist mehrere Spaltöfen auf. Liegt nur eine Ofenzelle vor, ist diese die Spalteinheit und somit der Spaltofen. Spaltöfen können zu Gruppen zusammengefasst werden, welche beispielsweise mit dem gleichen Einsatz versorgt werden. Die Spaltbedingungen innerhalb einer Ofengruppe werden in der Regel gleich oder ähnlich eingestellt werden.

Bei der thermischen Spaltung von Kohlenwasserstoffen üblicher Zusammensetzung, wie beispielsweise Naphtha, unter milden Spaltbedingungen entsteht eine sehr große Mengen an Pyrolysebenzin, welches aufgrund der großen Menge sehr schwierig in der Handhabung wird. Dies ist ein Resultat der vergleichsweise geringeren Umsetzung des Einsatzes im Spaltofen bei milden Spaltbedingungen. Milde Spaltbedingungen sind jedoch wünschenswert, da bei einer Spaltung unter milden Bedingungen ein größeres Verhältnis von Propylen zu Ethylen vorliegt als bei einer Spaltung unter normalen Spaltbedingungen, wie sie üblicherweise verwendet werden.

Mit dem erfindungsgemäßen Verfahren wird es möglich den zweiten Spaltofen unter milden Spaltbedingungen zu betreiben, da Einsatz und Spaltbedingungen aneinander angepasst sind. Nur durch die Anpassung von Einsatz und Spaltbedingungen ist es möglich, die um vorigen Absatz beschrieben Nachteile zu umgehen. Diese Nachteile und die aufgezeigte Lösung wurden im Rahmen der Erfindung erkannt.

Mit dem erfindungsgemäßen Verfahren wird es somit möglich, eine Anlage zum Steamcracken derartig zu betreiben, dass im Verhältnis zum Frischeinsatz mehr Propylen entsteht als in einer herkömmlichen Anlage, in welcher das erfindungsgemäße Verfahren nicht eingesetzt wird.

Je höher das Verhältnis von Propylen zu Ethylen für die Spaltbedingungen im zweiten Spaltofen gewählt wird, desto mehr Propylen entsteht im Verhältnis zum Frischeinsatz. Dies ist im Rahmen der Erfindung von Vorteil. Ein höheres Verhältnis von Propylen zu Ethylen geht jedoch mit einer geringeren Umsetzung des Einsatzes einher, so dass für die Werte nach oben hin technische und wirtschaftliche Grenzen auftreten. Innerhalb der in den Ansprüchen angegebenen Grenzwerte ist es gewährleistet, dass sich einerseits die erfinderischen Vorteile einstellen und anderseits der Steamcracker technisch beherrschbar und wirtschaftlich betreibbar ist.

Für die angegebenen Grenzwerte für die Spaltbedingungen im ersten Spaltofen gilt, dass innerhalb dieser ein technisch und wirtschaftlich vorteilhaftes Dampfspalten möglich ist, bei welchem Ethylen und Propylen als primäre Wertprodukte entstehen.

Mit Vorteil wird der zweite Kohlenwasserstoffeinsatz im zweiten Spaltofen mit Spaltbedingungen umgesetzt, die zu einem Verhältnis von Propylen zu Ethylen bis 1,4 kg/kg, besonders bevorzugt von 0,85 bis 1,2 kg/kg am Spaltofenaustritt führen.

Mit Vorteil wird der erste Kohlenwasserstoffeinsatz im ersten Spaltofen mit Spaltbedingungen umgesetzt, die zu einem Verhältnis von Propylen zu Ethylen von 0,3 bis 0,75 kg/kg, besonders bevorzugt von 0,4 bis 0,65 kg/kg am Spaltofenaustritt führen.

Insbesondere liegen die Werte für das Verhältnis von Propylen zu Ethylen für ersten und zweiten Kohlenwasserstoff um mindestens 0,1 kg/kg, vorzugsweise um mindestens 0,15 kg/kg, besonders bevorzugt um mindestens 0,2 kg/kg auseinander, damit sich die Vorteile der Erfindung in besonderem Maße einstellen. Erfindungsgemäß enthält der zweite Kohlenwasserstoffeinsatz überwiegend Kohlenwasserstoffe, die maximal eine Kohlenstoffzahl von 5 aufweisen. Ein solcher Kohlenwasserstoffeinsatz ist besonders geeignet für eine Spaltung bei milden Bedingungen. In ganz besonderem Maße gilt dies, wenn der zweite Kohlenwasserstoffeinsatz größtenteils aus Kohlenwasserstoffen besteht, die eine Kohlenstoffzahl von 5 oder/und 4 aufweisen.

Der Begriff "überwiegend" wird im Rahmen dieser Anmeldung verwendet, um deutlich zu machen, dass der Einsatz oder die Fraktion nicht ausschließlich aus Kohlenwasserstoffen mit der angegeben Kohlenstoffzahl besteht, sondern neben den Kohlenwasserstoffen der angegeben Kohlenstoffzahl auch Kohlenwasserstoffe mit anderen Kohlenstoffzahlen sowie andere Verunreinigungen vorhanden sein können. Bei der Trennung und Aufarbeitung des Frischeinsatzes, des Produktstroms und/oder den Fraktionen bleiben stets Reste der Komponente(n) in dem Produktstrom beziehungsweise in der Fraktion. Auch andere Verunreinigungen bleiben bestehen, so dass ein bearbeiteter Produkt- oder Fraktionsstrom stets Rückstände enthält. Da der Aufwand für Abtrennung und Aufarbeitung mit der zu erzielenden Reinheit extrem stark ansteigt, hängt es von wirtschaftlichen Faktoren ab, welchen Anteil an Rückständen in einem Strom enthalten sein dürfen. Wie hoch dieser Anteil ist, muss nach wirtschaftlichen Gesichtspunkten abgewogen werden. Als groben Richtwert für den Anteil an unerwünschten Kohlenwasserstoffen und anderen Verunreinigungen wird in der Regel gelten, dass diese mit maximal 30 bis 40 Gewichtsprozent in dem Produktstrom und/oder in der Fraktion enthalten sein dürfen. Meist wird sogar ein Maximalwert von 15 Gewichtsprozent oder weniger erreicht. Für den Kohlenwasserstoffeinsatz gilt daher, dass dieser die gewünschten Kohlenwasserstoffe mit mindestens 60 Gewichtsprozent, bevorzugt mindestens 80 Gewichtsprozent und weiter bevorzugt mindestens 90 Gewichtsprozent und besonders bevorzugt mindestens 95 Gewichtsprozent und ganz besonders bevorzugt mindestens 98 Gewichtsprozent enthalten. Dies gilt sowohl für den Frischeinsatz, wie für aus der Frischeinsatz-Fraktionierung gewonnenen Einsatz als auch für Einsatz aus rückgeführten Komponenten.

In besonders vorteilhafter Ausgestaltung der Erfindung wird dem zweiten Spaltofen eine oder mehrere rückgeführten Fraktionen, welche aus dem Produktstrom gewonnen werden und welche überwiegend Kohlenwasserstoffe enthält, die eine Kohlenstoffzahl von maximal 5 aufweisen, zugeführt. Der zweite Kohlenwasserstoffeinsatz enthält somit rückgeführte Fraktionen. Durch ein Rückführen solcher Fraktionen erhöht sich die Menge an geeigneten Einsatz für den zweiten Spaltofen beziehungsweise stellt eine solche Fraktion einen geeigneten zweiten Kohlenwasserstoffeinsatz für den zweiten Spaltofen dar. Auch wird eine Fraktion mit Kohlenwasserstoffen mit einer Kohlenstoffzahl von 4 sowie eine Fraktion mit einer Kohlenstoffzahl von 5 bei der Aufarbeitung des Produktstrom in Steamcracker gewonnen, welche nach der Abtrennung der Wertprodukte direkt oder nach weiteren Behandlungsschritten rückgeführt werden können.

Weiterhin ist es von Vorteil dem ersten Spaltofen zumindest eine aus dem Produktstrom abgetrennte und rückgeführte Fraktion, welche überwiegend Kohlenwasserstoffe mit einer Kohlenstoffzahl von zumindest 6 aufweist, zugeführt wird. Eine solche Fraktion ist als erster Kohlenwasserstoffeinsatz für den ersten Spaltofen geeignet.

Mit besonderem Vorteil wird ein Frischeinsatz verwendet, der in mindestens eine erste und eine zweite Frischeinsatz-Fraktion fraktioniert wird und die erste Frischeinsatz-Fraktion zumindest teilweise in den ersten Spaltofen und die zweite Frischeinsatz-Fraktion zumindest teilweise in den zweiten Spaltofen geführt wird. Durch eine Fraktionierung des Frischeinsatzes lässt sich erreichen, dass insbesondere für den zweiten Spaltofen ein Einsatz zur Verfügung steht, mit welchem sich die erfindungsgemäßen Vorteile in herausragender Weise einstellen. Dabei weisen erste und zweite Frischeinsatz-Fraktion eine unterschiedliche Zusammensetzung auf. Damit wird betont, dass es sich bei der Teilung des Frischeinsatzes um eine Fraktionierung handelt und nicht um eine einfache Aufteilung in zwei Mengen. Bei einer Fraktionierung wird nach unterschiedlichen Komponenten getrennt. Nach der Fraktionierung finden sich also einige Komponenten des Frischeinsatzes überwiegend in der ersten Frischeinsatz-Fraktion und andere Komponenten des Frischeinsatzes finden sich überwiegend in der zweiten Frischeinsatz-Fraktion.

In einer weiteren vorteilhaften Ausgestaltung der Erfindung wird dem zweiten Spaltofen ein Frischeinsatz zugeführt, welcher überwiegend aus Kohlenwasserstoffen besteht, die maximal eine Kohlenstoffzahl von 5 aufweisen. Ein solcher Frischeinsatz kann beispielsweise in einer Raffinerie oder bei der Erdgasgewinnung anfallen. Aufgrund seiner Beschaffenheit ist er als Einsatz im zweiten Spaltofen bei milden Spaltbedingungen sehr gut geeignet.

An dieser Stelle soll nochmals betont werden, dass sich die vorgenannten Einsätze rückgeführte Fraktionen, Frischeinsatz-Fraktion und Frischeinsätze aus Kohlenwasserstoffen mit einer Kohlenstoffzahl von maximal 5 als Einsatz für den zweiten Spaltofen eignen, da sie alle hervorragend für eine milde Spaltung geeignet sind. Um zu den Vorteilen der Erfindung zu gelangen, können die hier vorgeschlagenen Einsätze einzeln oder als Gemisch in den zweiten Spaltofen geführt werden. Als zweiter Kohlenwasserstoffeinsatz kann somit eine oder mehrere rückgeführte Fraktionen oder eine Frischeinsatz-Fraktion oder ein anderer Einsatz aus Kohlenwasserstoffen mit einer Kohlenstoffzahl von maximal 5 verwendet werden. Auch können rückgeführte Fraktion(en) und eine Frischeinsatz-Fraktion oder rückgeführte Fraktion(en) und ein anderer Einsatz aus Kohlenwasserstoffen mit einer Kohlenstoffzahl von maximal 5 oder eine Frischeinsatz-Fraktion und ein anderer Einsatz aus Kohlenwasserstoffen mit einer Kohlenstoffzahl von maximal 5 oder eine Mischung aus allen möglichen Einsätzen als zweiter Kohlenwasserstoffeinsatz verwendet werden.

Wie eingangs erläutert, ergibt sich das Verhältnis von Propylen zu Ethylen beim thermischen Dampfspalten aus einer Reihe unterschiedlicher Einflussfaktoren, bei denen die Spaltofenaustrittstemperatur, d.h. die Temperatur eines Produktstroms beim Verlassen der verwendeten Reaktorschlange (engl. Coil Output Temperature), eine wichtige Rolle spielt. Die Spaltofenaustrittstemperatur für die Umsetzung im der zweiten Spaltofen liegt vorteilhafterweise zwischen 680 °C und 820 °C, bevorzugt zwischen 700 °C und 800 °C und weiter bevorzugt zwischen 710 °C und 780°C und besonders bevorzugt zwischen 720 °C und 760 °C während die die Spaltofenaustrittstemperatur für die Umsetzung im ersten Spaltofen zwischen vorteilhafterweise zwischen 800 °C und 1000 °C, bevorzugt zwischen 820 °C und 950 °C und besonders bevorzugt zwischen 840 °C und 900 °C liegt. Dabei ist die Spaltofenaustrittstemperatur am ersten Spaltofen stets höher als am zweiten Spaltofen.

Dabei liegt die Spaltofenaustrittstemperatur für die Umsetzung im ersten Spaltofen bevorzugt um mindestens 10 °C, besonders bevorzugt um mindestens 15 °C und ganz besonders bevorzugt um mindestens 20 °C über der Spaltofenaustrittstemperatur für die Umsetzung im zweiten Spaltofen.

Im zweiten Spaltofen kann ferner eine geringere Dampfverdünnung als im ersten verwendet werden. Dies verringert die notwendige Verdünnungsdampfmenge und spart Energie ein. Eine geringere Dampfverdünnung im zweiten Spaltofen ist jedoch nicht notwendig, damit sich die wesentlichen Vorteil der Erfindung zeigen. Vorteilhafterweise werden im zweiten Spaltofen 0,15 bis 0,8 kg Wasserdampf pro kg Kohlenwasserstoff im Einsatz verwendet während im ersten Spaltofen 0,3 bis 1,5 kg Wasserdampf pro kg Kohlenwasserstoff im Einsatz verwendet werden.

Auch können mit Vorteil in dem Produktstrom enthaltene, insbesondere gesättigte Kohlenwasserstoffe mit einer Kohlenstoffzahl von 2 bis 3 mit Vorteil mittels thermischen Dampfspalten in einem Spaltofen für gasförmigen Einsatz umgesetzt werden. Dazu werden die gesättigten gasförmigen Kohlenwasserstoffe aus dem Produktstrom gewonnen und in den Spaltofen für gasförmigen Einsatz rückgeführt und dort umgesetzt.

Als Frischeinsatz für den ersten Kohlenwasserstoffeinsatz oder/und als Frischeinsatz zur Frischeinsatz-Fraktionierung können sowohl Gase oder Gasfraktionen wie Ethan, Propan oder Butan und entsprechende Gemische und Kondensate als auch flüssige Kohlenwasserstoffe und Kohlenwasserstoffgemische verwendet werden. Die genannten Gasgemische und Kondensate umfassen insbesondere sogenannte Erdgaskondensate (engl. Natural Gas Liquids, NGL). Die flüssigen Kohlenwasserstoffe und Kohlenwasserstoffgemische können beispielsweise aus der sogenannten Benzinfraktion von Rohöl stammen. Bei derartigen Rohbenzinen bzw. Naphthas (NT) und Kerosin handelt es sich um Gemische aus vorzugsweise gesättigten Verbindungen mit Siedepunkten zwischen 35 und 210 °C. Die Erfindung ist jedoch auch vorteilhaft beim Einsatz von Mitteldestillaten, atmosphärischen Rückständen und/oder hiervon abgeleiteten Gemischen aus der Rohölverarbeitung. Bei Mitteldestillaten handelt es sich um sogenannte leichte und schwere Gasöle, die als Ausgangsmaterialien zur Herstellung von leichten Heiz- und Dieselölen sowie von schwerem Heizöl verwendet werden können. Die enthaltenen Verbindungen weisen Siedepunkte von 180 bis 360 °C auf. Vorzugsweise handelt es sich um überwiegend gesättigte Verbindungen, die beim thermischen Dampfspalten umgesetzt werden können. Weiterhin können auch durch bekannte destillative Trennverfahren gewonnenen Fraktionen und entsprechenden Rückständen, aber auch die Verwendung jeweils hiervon, beispielsweise durch Hydrieren (engl. Hydrotreating) oder Hydrocracken, abgeleiteter Fraktionen verwendet werden. Beispiele sind Leicht-, Schwer- und Vakuumgasöl (engl. Atmospheric Gas Oil, AGO, bzw. Vacuum Gas Oil, VGO) sowie durch die genannten Hydrierverfahren behandelte Gemische und/oder Rückstände (engl. Hydrotreated Vacuum Gas Oil, HVGO, Hydrocracker Residue, HCR, bzw. Unconverted Oil, UCO).

Als Frischeinsatz für den ersten Kohlenwasserstoffeinsatz sind flüssige Kohlenwasserstoffe ganz besonders von Vorteil. Insbesondere werden als Frischeinsatz Erdgaskondensate und/oder Rohölfraktionen und/oder hiervon abgeleiteten Gemische verwendet.

Vorteilhafterweise umfasst die Erfindung damit die Verwendung von Kohlenwasserstoffgemischen mit einem Siedebereich von bis zu 600 °C als erstem Kohlenwasserstoffeinsatz als Frischeinsatz für den ersten Kohlenwasserstoffeinsatz. Innerhalb dieses Gesamtbereichs können auch Kohlenwasserstoffgemische mit abweichenden Siedebereichen verwendet werden, beispielsweise mit Siedebereichen von bis zu 360 °C oder von bis zu 240 °C. Die Reaktionsbedingungen im Spaltofen werden hierbei auf die jeweils eingesetzten Kohlenwasserstoffgemische abgestimmt.

So kann die Erfindung mit Vorteil jedoch auch mit beliebigen anderen Frischeinsätzen verwendet werden, die vergleichbare Eigenschaften aufweisen, wie beispielsweise biogene oder/und synthetische Kohlenwasserstoffe.

### Kurze Beschreibung der Zeichnung

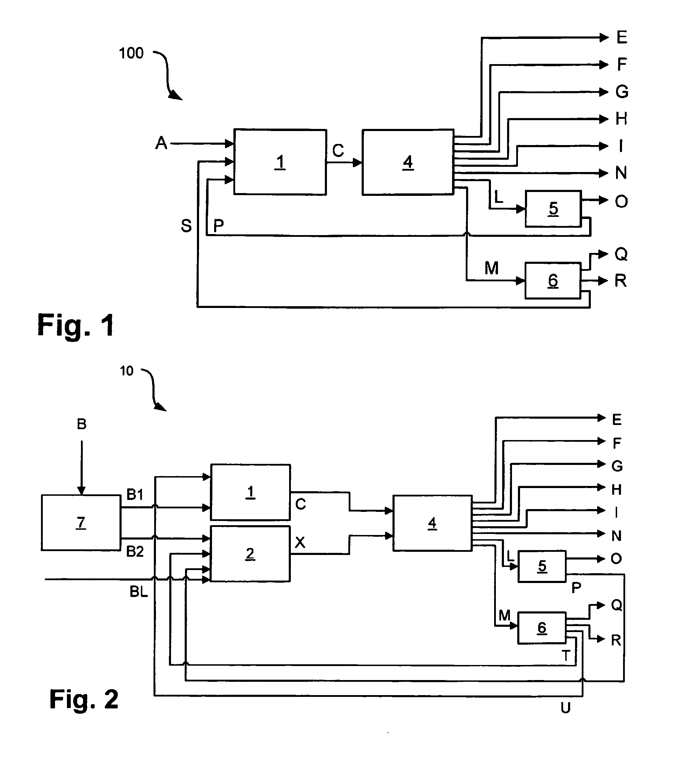

Das erfindungsgemäße Verfahren in besonders vorteilhafter Ausgestaltung soll anhand der Prozessschaubilder, welche die wesentlichen Prozessschritte schematisch zeigen, näher erklärt werden. Zum besseren Verständnis wird zuerst anhand von Figur 1 das bekannte Verfahren dargelegt.

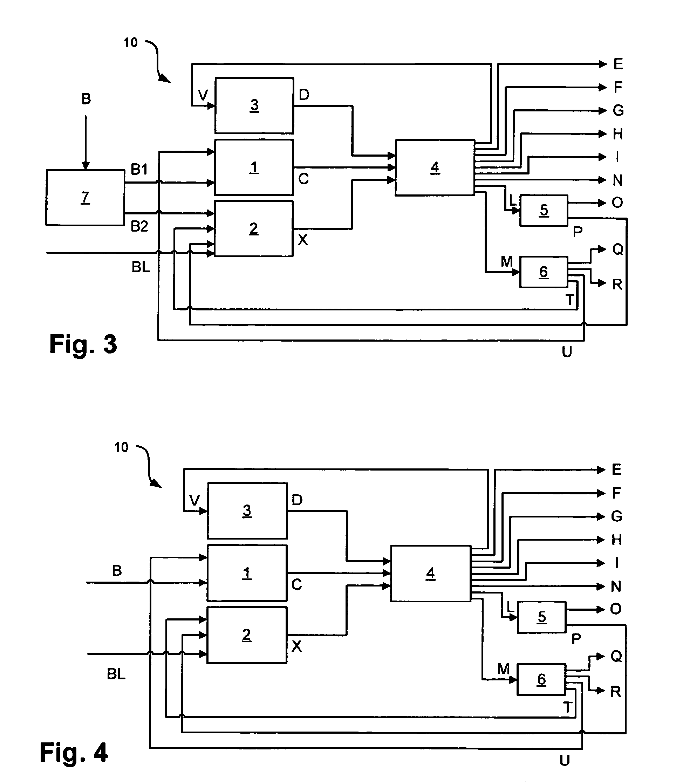

Figur 1 zeigt dazu in schematischer Darstellung ein bekanntes Vorgehen zur Olefinherstellung. Figur 2 zeigt in schematischer Darstellung die wesentlichen Schritte des erfindungsgemäßen Verfahrens in besonders vorteilhafter Ausgestaltung und Figur 3 und 4 zeigen, ebenfalls schematisch, die wesentlichen Schritte einer besonders vorteilhaften Ausgestaltung der Erfindung. In den Figuren tragen einander entsprechende Elemente identische Bezugzeichen.

Das schematische Prozessschaubild 100 der Figur 1 für das bekannte Verfahren beinhaltet einen Spaltofen 1, in welchen der Frischeinsatz A (beispielsweise Naphtha) sowie die rückgeführten Fraktionen S und P als Kohlenwasserstoffeinsatz geführt werden. Im Spaltofen 1 wird der Kohlenwasserstoffeinsatz in Konvektions- und Strahlungszone erwärmt und umgesetzt. In den Spaltofen wird Wasserdampf zugegeben, meist 0,5 bis 1 kg Prozessdampf pro kg Kohlenwasserstoff. Aus dem Spaltofen 1 tritt ein Produktstrom C aus, der direkt am Austritt aus dem Spaltofen auch als Spaltproduktstrom bezeichnet wird. Beim Austritt aus dem Spaltofen weist dieser Spaltproduktstrom eine Temperatur aus, die normalerweise zwischen 840 °C und 900°C liegt. Das Verhältnis von Propylen zu Ethylen liegt in der Regel bei 0,35 bis 0,6 kg/kg. Nach einem ersten Quenchen (nicht dargestellt) wird der Produktstrom wird in einer Aufarbeitungseinheit (englisch: processing unit) 4 verarbeitet. Aus der Aufarbeitungseinheit werden als wesentliche Produktfraktionen E bis N folgende Fraktionen gewonnen: Wasserstoff E, Ablauge F, Methan G, Ethylen H, Propylen I, gasförmige Kohlenwasserstoffe L mit einer Kohlenstoffzahl von 4, Pyrolysebenzin M und Pyrolyseöl N. Die gasförmigen Kohlenwasserstoffe L mit einer Kohlenstoffzahl von 4 werden in einer C4-Aufarbeitungseinheit 5, welche für die Verarbeitung von Kohlenwasserstoffen mit einer Kohlenstoffzahl von 4 benutzt wird, weiter behandelt. Eine solche C4-Aufarbeitungseinheit 5 behandelt die Fraktion mit einer Kohlenstoffzahl von 4 derartig weiter, dass Butadien O abgeführt werden können. Die übrigen Kohlenwasserstoff mit einer Kohlenstoffzahl von 4 stellen eine Fraktion P dar, die in den Spaltofen 1 rückgeführt wird. Das Pyrolysebenzin M, welches Kohlenwasserstoffe mit einer Kohlenstoffzahl von 5 und mehr umfasst, wird in einer Pyrolysebenzin-Aufarbeitungseinheit 6 weiterverarbeitet und es werden Aromate Q und Kohlenwasserstoffe R mit einer Kohlenstoffzahl von beispielsweise mehr als 9 abgeführt. Die übrigen Kohlenwasserstoffe mit einer Kohlenstoffzahl von 5 und mehr werden als Fraktion S in den Spaltofen 1 rückgeführt. Die Aufarbeitungseinheit 4 sowie die C4-Aufarbeitungseinheit 5 und die Pyrolysebenzin-Aufarbeitungseinheit 6 umfassen übliche Einheiten zur Weiterverarbeitung des Produktstroms beziehungsweise der Produktfraktionen, welche zur Ausführung verschiedener Prozessschritte dienen, wie beispielsweise Verdichtung, Kondensation und Abkühlung, Trocknung, Destillation und Fraktionierung, Extraktion und Hydrierung. Die Prozessschritte sind in Olefinanlagen üblich und dem Fachmann bekannt.

Das schematische Prozessschaubild 10 der Figur 2 zeigt nun das erfindungsgemäße Verfahren in einer besonders vorteilhaften Ausgestaltung und seine wesentlichen Prozessschritte. Zusätzlich zu dem Spaltofen 1 ist hier ein zweiter Spaltofen 2 vorhanden sowie eine Frischeinsatz-Fraktionierungseinheit 7. Ein Frischeinsatz B (beispielsweise Naphtha) wird nun in der Frischeinsatz-Fraktionierungseinheit 7 fraktioniert und die erste Frischeinsatz-Fraktion B1 wird in den ersten Spaltofen 1 geführt, während die zweite Frischeinsatz-Fraktion B2 in den zweiten Spaltofen 2 geführt wird. Für die Prozesse zur Fraktionierung des Frischeinsatzes werden die üblichen Methoden zur Separation und Behandlung von Kohlenwasserstoffströmen verwendet, wie sie aus Olefinanlagen aus Raffinerien bekannt sind. Diese kennt der Fachmann und er weiß sie einzusetzen. In den ersten Spaltofen 1 werden zusätzlich eine Fraktion U und in den zweiten Spaltofen 2 zusätzlich die Fraktionen T und P rückgeführt (näheres siehe weiter unten). Weiterhin wird dem zweiten Spaltofen ein weiterer Einsatz BL aus Kohlenwasserstoffen mit einer Kohlenstoffzahl von maximal 5 als Frischeinsatz zugeführt. Aus dem ersten Spaltofen 1 tritt wiederum der Spaltproduktstrom C mit den oben genannten Eigenschaften aus. Aus dem zweiten Spaltofen 2 tritt der Spaltproduktstrom X aus. Der Spaltproduktstrom X weist eine Temperatur aus, die vorteilhafterweise zwischen 700 °C und 800°C liegt. Das Verhältnis von Propylen zu Ethylen liegt dabei vorteilhafterweise zwischen 0,85 bis 1,5 kg/kg. Die Produktströme C und X werden in der Aufarbeitungseinheit 4 weiterverarbeitet und an geeigneter Stelle zu einem gemeinsamen Produktstrom zusammengeführt. Die Prozesse zur Weiterbehandlung und Aufarbeitung in der Aufarbeitungseinheit 4 sind bekannt und wurden eben beschrieben. So führt die Aufarbeitungseinheit 4 auch, wie eben beschrieben, zu den Produktfraktionen E bis N. Auch die Produktfraktionen L und M werden, wie eben beschrieben, in den speziellen Aufarbeitungseinheiten 5 und 6 weiterbehandelt. Im Gegensatz zu dem in Figur 1 beschrieben Verfahren wird nun vorteilhafterweise auch die Fraktion P, welche Kohlenwasserstoffe mit einer Kohlenstoffzahl von 4 enthält, nicht in den Spaltofen 1 sondern in den zweiten Spaltofen 2 rückgeführt. In der Pyrolysebenzin-Aufarbeitungseinheit 6 werden neben den oben erwähnten Fraktionen Q und R die Fraktionen T und U gewonnen. Die Fraktion T, welche Kohlenwasserstoffe mit einer Kohlenstoffzahl von 5 enthält, wird vorteilhafterwiese in den zweiten Spaltofen 2 rückgeführt, während die Fraktion U, welche Kohlenwasserstoffe mit einer Kohlenstoffzahl von 6 und mehr, insbesondere zwischen 6 und 9, enthält, vorteilhafterweise in den ersten Spaltofen 1 rückgeführt wird. In Figur 2 werden verschiedene Einsätze für den zweiten Spaltofen geführt. Diese bilden dann den zweiten Kohlenwasserstoffeinsatz. Es soll erwähnt werden, dass die Aufzählung der verschiedenen Einsätze nicht abschließend ist und insbesondere, dass die in Figur 2 gezeigten Einsätze für den zweiten Spaltofen B2, BL, T und P nicht immer alle in den zweiten Spaltofen 2 geführt werden müssen, sondern dass es in vielen Fällen genügt, einen Teil der möglichen Einsätze in den zweiten Spaltofen 2 zu führen, beispielsweise eine rückgeführte Fraktion T aus Kohlenwasserstoffen mit einer Kohlenstoffzahl von 5 und ein Frischeinsatz BL aus Kohlenwasserstoffen mit einer Kohlenstoffzahl von maximal 5 oder beispielsweise rückgeführte Fraktionen T und P mit Kohlenwasserstoffen mit Kohlenstoffzahlen von 5 und 4 und LPG BL. Kurz gesagt sind folgende Einsätze in den zweiten Spaltofen möglich: B2, BL, T, P, B2+BL, B2+T, B2+P, BL+T, BL+P, T+P, B2+BL+T, B2+BL+P, B2+P+T, BL+P+T oder B2+BL+P+T.

Ebenfalls eine besonders vorteilhafte Ausgestaltung der Erfindung beinhaltet Figur 3. Figur 3 weist das gleiche schematische Prozessschaubild auf, wie es auch Figur 2 zeigt. Ergänzt ist dieses um einen Spaltofen 3 für gasförmigen Einsatz, in welche eine Fraktion V als Einsatz geführt wird. Die Fraktion V enthält gesättigte gasförmige Kohlenwasserstoffe mit einer Kohlenstoffzahl von 2 oder 3, welche ebenfalls in der Aufarbeitungseinheit 4 gewonnen werden.

Auch in Figur 4 wird eine vorteilhafte Ausgestaltung der Erfindung gezeigt. Figur 4 beinhaltet das gleiche schematische Prozessschaubild auf wie Figur 2, jedoch fehlt hier die Frischeinsatz-Fraktionierung. Frischeinsatz wird hier als Frischeinsatz B dem ersten Spaltofen 1 zugegeben und dem zweiten Spaltofen 2 wird ein Frischeinsatz BL aus Kohlenwasserstoffen mit einer Kohlenstoffzahl von maximal 5 zugegeben. Die weiteren Verfahrenschritte wurden bereits in der Figurenbeschreibung zu Figur 2 erläutert.

### Bezugszeichenliste

- 1: Spaltofen (normale Spaltbedingungen)
- 2: Spaltofen (milde Spaltbedingungen)
- 3: Spaltofen für gasförmigen Einsatz
- 4: Aufarbeitungseinheit
- 5: C4-Aufarbeitungseinheit
- 6: Pyrolysebenzin-Aufarbeitungseinheit
- 7: Frischeinsatz-Fraktionierungseinheit

- 10: schematische Prozessschaubilder für ein bekanntes Verfahren

- 100: schematische Prozessschaubilder für das erfindungsgemäße Verfahren in einer besonders vorteilhaften Ausgestaltung

- A, B, BL: Frischeinsatz
- B1, B2: Frischeinsatz-Fraktionen
- C, D, X: Produktströme
- E-V: Produktfraktionen

## Patentansprüche

1. Verfahren zur Umsetzung von Kohlenwasserstoffeinsätzen durch thermisches Dampfspalten zu einem olefinhaltigen Produktstrom, welcher zumindest Ethylen und Propylen enthält, wobei ein erster Kohlenwasserstoffeinsatz in mindestens einem ersten Spaltofen (1) und ein zweiter Kohlenwasserstoffeinsatz in mindesten einem zweiten Spaltofen (2) wenigstens teilweise umgesetzt wird, **dadurch gekennzeichnet, dass** der zweite Kohlenwasserstoffeinsatz im zweiten Spaltofen (2) mit Spaltbedingungen umgesetzt wird, die zu einem Verhältnis von Propylen zu Ethylen von 0,85 bis 1,6 kg/kg am Spaltofenaustritt führen, und dass der erste Kohlenwasserstoffeinsatz im ersten Spaltofen (1) mit Spaltbedingungen umgesetzt wird, die zu einem Verhältnis von Propylen zu Ethylen von 0,25 bis 0,85 kg/kg am Spaltofenaustritt führen, wobei der Wert für das Verhältnis von Propylen zu Ethylen für den zweiten Kohlenwasserstoffeinsatz über dem Wert für das Verhältnis von Propylen zu Ethylen für den ersten Kohlenwasserstoffeinsatz liegt und der zweite Kohlenwasserstoffeinsatz überwiegend Kohlenwasserstoffe enthält, die maximal eine Kohlenstoffzahl von 5 aufweisen.

2. Verfahren nach Anspruch 1, **dadurch gekennzeichnet, dass** der zweite Kohlenwasserstoff im zweiten Spaltofen (2) mit Spaltbedingungen umgesetzt wird, die zu einem Verhältnis von Propylen zu Ethylen bis 1,2 kg/kg am Spaltofenaustritt führen.

3. Verfahren nach Anspruch 1 oder 2, **dadurch gekennzeichnet dass** der der erste Kohlenwasserstoffeinsatz im ersten Spaltofen (1) mit Spaltbedingungen umgesetzt wird, die zu einem Verhältnis von Propylen zu Ethylen von 0,3 bis 0,75 kg/kg, besonders bevorzugt von 0,4 bis 0,65 kg/kg am Spaltofenaustritt führen.

4. Verfahren nach einem der Ansprüche 1 bis 3, bei dem die Werte für das Verhältnis von Propylen zu Ethylen für ersten und zweiten Kohlenwasserstoff um mindestens 0,1 kg/kg, vorzugsweise um mindestens 0,15 kg/kg, besonders bevorzugt um mindestens 0,2 kg/kg auseinander liegen.

5. Verfahren nach einem der Ansprüche 1 bis 4, **dadurch gekennzeichnet, dass** der zweite Kohlenwasserstoffeinsatz größtenteils aus Kohlenwasserstoffen besteht, die eine Kohlenstoffzahl von 5 oder/und 4 aufweisen.

6. Verfahren nach einem der Ansprüche 1 bis 5, **dadurch gekennzeichnet, dass** dem zweiten Spaltofen eine oder mehrere rückgeführten Fraktionen (P, T), welche aus dem Produktstrom gewonnen werden und welche überwiegend Kohlenwasserstoffe enthält, die eine Kohlenstoffzahl von maximal 5 aufweisen, zugeführt wird.

7. Verfahren nach einem der Ansprüche 1 bis 6, **dadurch gekennzeichnet, dass** dem ersten Spaltofen zumindest eine aus dem Produktstrom abgetrennte und rückgeführte Fraktion (U), welche überwiegend Kohlenwasserstoffe mit einer Kohlenstoffzahl von zumindest 6 aufweist, zugeführt wird.

8. Verfahren nach einem der Ansprüche 1 bis 7, **dadurch gekennzeichnet, dass** ein Frischeinsatz verwendet wird, der in mindestens eine erste und eine zweite Frischeinsatz-Fraktion fraktioniert wird und die erste Frischeinsatz-Fraktion zumindest teilweise in den ersten Spaltofen und die zweite Frischeinsatz-Fraktion zumindest teilweise in den zweiten Spaltofen geführt wird.

9. Verfahren nach einem der Ansprüche 1 bis 8, **dadurch gekennzeichnet, dass** dem zweiten Spaltofen (2) neben der zweiten Frischeinsatz-Fraktion (B2) ein weiterer Frischeinsatz (BL) zugeführt wird, welcher überwiegend aus Kohlenwasserstoffen besteht, die maximal eine Kohlenstoffzahl von 5 aufweisen.

10. Verfahren nach einem der Ansprüche 1 bis 9, bei welchem die Spaltofenaustrittstemperatur für die Umsetzung im zweiten Spaltofen (2) zwischen 680 °C und 820 °C, bevorzugt zwischen 700 °C und 800 °C und weiter bevorzugt zwischen 710 °C und 780°C und besonders bevorzugt zwischen 720 °C und 760 °C liegt und die Spaltofenaustrittstemperatur für die Umsetzung im ersten Spaltofen (1) zwischen 800 °C und 1000 °C, bevorzugt zwischen 820 °C und 950 °C und besonders bevorzugt zwischen 840 °C und 900 °C liegt, wobei die Spaltofenaustrittstemperatur des ersten Spaltofens (1) über der des zweiten Spaltofens (2) liegt.

11. Verfahren nach Anspruch 10, bei welchem die Spaltofenaustrittstemperatur für die Umsetzung im ersten Spaltofen (1) um mindestens 10 °C, bevorzugt um mindestens 15°C, besonders bevorzugt um mindestens 20 °C über der Spaltofenaustrittstemperatur für die Umsetzung im zweiten Spaltofen (2) liegt.

12. Verfahren nach einem der Ansprüche 1 bis 11, bei welchem im ersten Spaltofen (1) 0,3 bis 1,5 kg Wasserdampf pro kg Kohlenwasserstoffeinsatz und im zweiten Spaltofen (2) 0,15 bis 0,8 kg Wasserdampf pro kg Kohlenwasserstoffeinsatz verwendet wird.

13. Verfahren nach einem der Ansprüche 1 bis 12, bei welchem aus dem Produktstrom zumindest eine Fraktion (V), welche überwiegend Kohlenwasserstoffe mit einer Kohlenstoffzahl von 2 oder 3 aufweist, gewonnen wird und in einem Spaltofen (3) für gasförmigen Einsatz zumindest teilweise umgesetzt wird.

14. Verfahren nach einem der Ansprüche 1 bis 13, **dadurch gekennzeichnet, dass** als Frischeinsatz (B) für den ersten Spaltofen (1) oder/und für den Frischeinsatz für die Frischeinsatz-Fraktionierung (7) Erdgaskondensate und/oder Rohölfraktionen, insbesondere Naphtha, und/oder synthetische und/oder biogene Kohlenwasserstoffe und/oder hiervon abgeleiteten Gemische verwendet werden.

## Claims

1. Process for converting hydrocarbon inputs by thermal steamcracking to an olefin-containing product stream comprising at least ethylene and propylene, with at least partial conversion of a first hydrocarbon input in at least one first cracking furnace (1) and of a second hydrocarbon input in at least one second cracking furnace (2), **characterized in that** the second hydrocarbon input is converted in the second cracking furnace (2) with cracking conditions that lead to a ratio of propylene to ethylene of 0.85 to 1.6 kg/kg at the cracking furnace exit, and **in that** the first hydrocarbon input is converted in the first cracking furnace (1) with cracking conditions that lead to a ratio of propylene to ethylene of 0.25 to 0.85 kg/kg at the cracking furnace exit, the value for the ratio of propylene to ethylene for the second hydrocarbon input being above the value for the ratio of propylene to ethylene for the first hydrocarbon input and the second hydrocarbon input comprising predominantly hydrocarbons having a maximum carbon number of 5.

2. Process according to Claim 1, **characterized in that** the second hydrocarbon input is converted in the second cracking furnace (2) with cracking conditions that lead to a ratio of propylene to ethylene to 1.2 kg/kg, at the cracking furnace exit.

3. Process according to Claim 1 or 2, **characterized in that** the first hydrocarbon input is converted in the first cracking furnace (1) with cracking conditions that lead to a ratio of propylene to ethylene of 0.3 to 0.75 kg/kg, more preferably of 0.4 to 0.65 kg/kg, at the cracking furnace exit.

4. Process according to any of Claims to 1 to 3, in which the values for the ratio of propylene to ethylene for the first and second hydrocarbon inputs differ by at least 0.1 kg/kg, preferably by at least 0.15 kg/kg, more preferably by at least 0.2 kg/kg.

5. Process according to any of Claims 1 to 4, **characterized in that** the second hydrocarbon input consists for the most part of hydrocarbons having a carbon number of 5 or/and 4.

6. Process according to any of Claims 1 to 5, **characterized in that** the second cracking furnace is supplied with one or more recycled fractions (P, T) which are obtained from the product stream and which comprise predominantly hydrocarbons having a carbon number of not more than 5.

7. Process according to any of Claims 1 to 6, **characterized in that** the first cracking furnace is supplied with at least one fraction (U) which has been separated from the product stream and recycled, comprising predominantly hydrocarbons having a carbon number of at least 6.

8. Process according to any of Claims 1 to 7, **characterized in that** a fresh input is used, which is fractionated into at least one first and one second fresh input fraction, and the first fresh input fraction is conducted at least partly into the first cracking furnace and the second fresh input fraction at least partly into the second cracking furnace.

9. Process according to any of Claims 1 to 8, **characterized in that** the second cracking furnace (2) is supplied not only with the second fresh input fraction (B2) but also with a further fresh input (BL) consisting predominantly of hydrocarbons having a maximum carbon number of 5.

10. Process according to any of Claims 1 to 9, in which the cracking furnace exit temperature for the conversion in the second cracking furnace (2) is between 680°C and 820°C, preferably between 700°C and 800°C and further preferably between 710°C and 780°C and more preferably between 720°C and 760°C, and the cracking furnace exit temperature for the conversion in the first cracking furnace (1) is between 800°C and 1000°C, preferably between 820°C and 950°C and more preferably between 840°C and 900°C, the cracking furnace exit temperature of the first cracking furnace (1) being above that of the second cracking furnace (2).

11. Process according to Claim 10, in which the cracking furnace exit temperature for the conversion in the first cracking furnace (1) is at least 10°C above, preferably at least 15°C above, more preferably at least 20°C above, the cracking furnace exit temperature for the conversion in the second cracking furnace (2).

12. Process according to any of Claims 1 to 11, in which 0.3 to 1.5 kg of steam per kg of hydrocarbon input is used in the first cracking furnace (1), and 0.15 to 0.8 kg of steam per kg of hydrocarbon input in the second cracking furnace (2).

13. Process according to any of Claims 1 to 12, in which at least one fraction (V) comprising predominantly hydrocarbons having a carbon number of 2 or 3 is obtained from the product stream and at least partly converted in a cracking furnace (3) for gaseous input.

14. Process according to any of Claims 1 to 13, **characterized in that** the fresh input (B) used for the first cracking furnace (1) or/and for the fresh input for the fresh input fractionation (7) comprises natural gas condensates and/or crude oil fractions, especially naphtha, and/or synthetic and/or biogenic hydrocarbons and/or mixtures derived therefrom.

## Revendications

1. Procédé de mise en réaction d'alimentations hydrocarbonées par clivage thermique à la vapeur en un courant de produits contenant des oléfines, qui contient au moins de l'éthylène et du propylène, une première alimentation hydrocarbonée étant au moins partiellement mise en réaction dans au moins un premier four de clivage (1) et une seconde alimentation hydrocarbonée dans au moins un second four de clivage (2), **caractérisé en ce que** la seconde alimentation hydrocarbonée est mise en réaction dans le second four de clivage (2) dans des conditions de clivage qui conduisent à un rapport entre le propylène et l'éthylène de 0,85 à 1,6 kg/kg à la sortie du four de clivage, et **en ce que** la première alimentation hydrocarbonée est mise en réaction dans le premier four de clivage (1) dans des conditions de clivage qui conduisent à un rapport entre le propylène et l'éthylène de 0,25 à 0,85 kg/kg à la sortie du four de clivage, la valeur du rapport entre le propylène et l'éthylène pour la seconde alimentation hydrocarbonée étant supérieure à la valeur du rapport entre le propylène et l'éthylène pour la première alimentation hydrocarbonée, et la seconde alimentation hydrocarbonée contenant principalement des hydrocarbures qui présentent au plus un nombre de carbones de 5.

2. Procédé selon la revendication 1, **caractérisé en ce que** la seconde alimentation hydrocarbonée est mise en réaction dans le second four de clivage (2) dans des conditions de clivage qui conduisent à un rapport entre le propylène et l'éthylène jusqu'à 1,2 kg/kg à la sortie du four de clivage.

3. Procédé selon la revendication 1 ou 2, **caractérisé en ce que** la première alimentation hydrocarbonée est mise en réaction dans le premier four de clivage (1) dans des conditions de clivage qui conduisent à un rapport entre le propylène et l'éthylène de 0,3 à 0,75 kg/kg, de manière particulièrement préférée de 0,4 à 0,65 kg/kg, à la sortie du four de clivage.

4. Procédé selon l'une quelconque des revendications 1 à 3, dans lequel les valeurs pour le rapport entre le propylène et l'éthylène pour la première et la seconde alimentation hydrocarbonée sont séparées l'une de l'autre d'au moins 0,1 kg/kg, de préférence d'au moins 0,15 kg/kg, de manière particulièrement préférée d'au moins 0,2 kg/kg.

5. Procédé selon l'une quelconque des revendications 1 à 4, **caractérisé en ce que** la seconde alimentation hydrocarbonée est constituée en majeure partie d'hydrocarbures qui présentent un nombre de carbones de 5 et/ou 4.

6. Procédé selon l'une quelconque des revendications 1 à 5, **caractérisé en ce qu'**une ou plusieurs fractions recyclées (P, T), qui sont obtenues à partir du courant de produits et qui contiennent principalement des hydrocarbures qui présentent un nombre de carbones d'au plus 5, sont introduites dans le second four de clivage.

7. Procédé selon l'une quelconque des revendications 1 à 6, **caractérisé en ce qu'**au moins une fraction séparée du courant de produits et recyclée (U), qui comprend principalement des hydrocarbures ayant un nombre de carbones d'au moins 6, est introduite dans le premier four de clivage.

8. Procédé selon l'une quelconque des revendications 1 à 7, **caractérisé en ce qu'**une alimentation fraîche, qui est fractionnée en au moins une première et une seconde fraction d'alimentation fraîche, est utilisée, et la première fraction d'alimentation fraîche est introduite au moins en partie dans le premier four de clivage et la seconde fraction d'alimentation fraîche est introduite au moins en partie dans le second four de clivage.

9. Procédé selon l'une quelconque des revendications 1 à 8, **caractérisé en ce qu'**en plus de la seconde fraction d'alimentation fraîche (B2), une alimentation fraîche supplémentaire(BL), qui est principalement constituée d'hydrocarbures présentant au plus un nombre de carbones de 5, est introduite dans le second four de clivage (2).

10. Procédé selon l'une quelconque des revendications 1 à 9, dans lequel la température de sortie du four de clivage pour la réaction dans le second four de clivage (2) est comprise entre 680 °C et 820 °C, de préférence entre 700 °C et 800 °C et de manière davantage préférée entre 710 °C et 780 °C et de manière particulièrement préférée entre 720 °C et 760 °C, et la température de sortie du four de clivage pour la réaction dans le premier four de clivage (1) est comprise entre 800 °C et 1 000 °C, de préférence entre 820 °C et 950 °C, et de manière particulièrement préférée entre 840 °C et 900 °C, la température de sortie du premier four de clivage (1) étant supérieure à celle du second four de clivage (2).

11. Procédé selon la revendication 10, dans lequel la température de sortie du four de clivage pour la réaction dans le premier four de clivage (1) est supérieure d'au moins 10 °C, de préférence d'au moins 15 °C, de manière particulièrement préférée d'au moins 20 °C, à la température de sortie du four de clivage pour la réaction dans le second four de clivage (2).

12. Procédé selon l'une quelconque des revendications 1 à 11, dans lequel 0,3 à 1,5 kg de vapeur d'eau par kg d'alimentation hydrocarbonée est utilisée dans le premier four de clivage (1) et 0,15 à 0,8 kg de vapeur d'eau par kg d'alimentation hydrocarbonée dans le second four de clivage (2).

13. Procédé selon l'une quelconque des revendications 1 à 12, dans lequel au moins une fraction (V), qui comprend principalement des hydrocarbures ayant un nombre de carbones de 2 ou 3, est obtenue à partir du courant de produits et mise en réaction au moins en partie dans un four de clivage (3) pour alimentation gazeuse.

14. Procédé selon l'une quelconque des revendications 1 à 13, **caractérisé en ce que** des condensats de gaz naturel et/ou des fractions de pétrole, notamment du naphta, et/ou des hydrocarbures synthétiques et/ou biogènes et/ou des mélanges, dérivés de ceux-ci, sont utilisés en tant qu'alimentation fraîche (B) pour le premier four de clivage (1) et/ou pour l'alimentation fraîche pour le fractionnement de l'alimentation fraîche (7).
